(19) Europäisches Patentamt
European Patent Office
Office européen des brevets

(11) Veröffentlichungsnummer : **0 473 626 B1**

# (12) EUROPÄISCHE PATENTSCHRIFT

(45) Veröffentlichungstag der Patentschrift : **01.12.93 Patentblatt 93/48**

(51) Int. Cl.$^5$ : **C07F 9/6506,** A61K 31/675, C07F 9/6503

(21) Anmeldenummer : **90907035.1**

(22) Anmeldetag : **18.05.90**

(86) Internationale Anmeldenummer : **PCT/EP90/00798**

(87) Internationale Veröffentlichungsnummer : **WO 90/14348 29.11.90 Gazette 90/27**

## (54) NEUE DIPHOSPHONSÄUREDERIVATE, VERFAHREN ZU DEREN HERSTELLUNG UND DIESE VERBINDUNGEN ENTHALTENDE ARZNEIMITTEL.

Die Akte enthält technische Angaben, die nach dem Eingang der Anmeldung eingereicht wurden und die nicht in dieser Patentschrift enthalten sind.

(30) Priorität : **26.05.89 DE 3917153**

(43) Veröffentlichungstag der Anmeldung : **11.03.92 Patentblatt 92/11**

(45) Bekanntmachung des Hinweises auf die Patenterteilung : **01.12.93 Patentblatt 93/48**

(84) Benannte Vertragsstaaten : **AT BE CH DE DK ES FR GB IT LI LU NL SE**

(56) Entgegenhaltungen :
**EP-A- 0 170 228**
**EP-A- 0 258 618**
**EP-A- 0 282 309**

(73) Patentinhaber : **BOEHRINGER MANNHEIM GMBH**
**D-68298 Mannheim (DE)**

(72) Erfinder : **BOSIES, Elmar**
**Delpstra e 11**
**D-6940 Weinheim (DE)**
Erfinder : **BAUSS, Frieder**
**Füllenweg 5**
**D-6715 Lambsheim (DE)**

(74) Vertreter : **Weber, Manfred, Dr. et al**
**c/o Boehringer Mannheim GmbH,**
**Patentabteilung, Sandhoferstrasse 116**
**D-68298 Mannheim (DE)**

EP 0 473 626 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue Diphosphonsäurederi- vate, Verfahren zu deren Herstellung sowie Arzneimittel, die diese Substanzen enthalten.

In der DE-PS 18 13 659 sind Diphosphonsäurederivate beschrieben, von denen die 1-Hydroxyethan-1,1-diphosphonsäure als Mittel zur Behandlung von Morbus Paget Bedeutung erlangt hat.

In der EP 274 158 sind 1.1-Diphosphonate beschrieben, deren Alkylkette durch einen Piperidinring substituiert und durch ein Heteroatom unterbrochen ist.

In der EP 186 405 sind weiterhin 1.1-Diphosphonsäuren beschrieben, deren Alkylkette durch Sechsring-Heterocyclen substituiert und durch ein Heteroatom unterbrochen ist.

Aus der EP-A-0 170 228 sind schließlich bereits 1,1-Diphosphonsäurederivate bekannt, die einen über eine Alkylenkette mit 2 bis 8 C-Atomen an den Diphosphonatrest gebundenen heterocyclischen Fünfring aufweisen und zur Behandlung von Calciumstoffwechselstörungen geeignet sind.

Es wurde nun gefunden, daß durch 5-Ring-Heterocyclen substituierte Alkan-1,1-diphosphonsäuren, bei denen die Alkylkette durch Sauerstoff unterbrochen ist, eine deutlich ausgeprägtere Wirkung auf den Calciumstoffwechsel zeigen als die bisher bekannten Verbindungen. Diese Substanzen sind somit besonders zu einer breiten Behandlung von Calciumstoffwechselstörungen geeignet. Sie lassen sich vor allem sehr gut dort einsetzen, wo der Knochenauf- und -abbau gestört ist, d.h. sie sind geeignet zur Behandlung von Erkrankungen des Skelettsystems wie z.B. Osteoporose, Morbus Paget, Morbus Bechterew u.a.. Aufgrund dieser Eigenschaften finden sie aber auch Verwendung in der Therapie von Knochenmetastasen, der Urolithiasis und zur Verhinderung heterotoper Ossifikationen. Durch ihre Beeinflussung des Calciumstoffwechsels bilden sie weiterhin eine Grundlage für die Behandlung der rheumatoiden Arthritis, der Osteoarthritis und der degenerativen Arthrose.

Gegenstand der vorliegenden Erfindung sind demnach Diphosphonate der allgemeinen Formel I

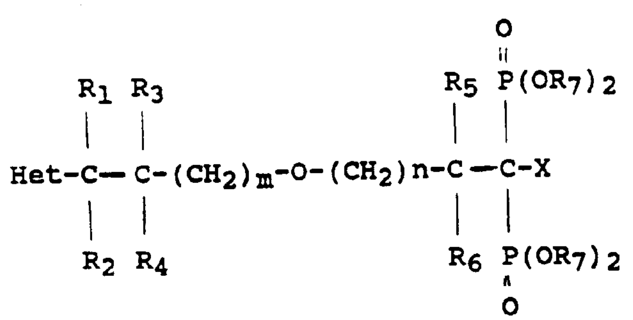

(I),

in der

| | |
|---|---|
| Het | einen Pyrazol-, Imidazol-, Thiazol-, Isothiazol-, Oxazol-, Isoxazol-, 1.2.3-Triazol-, 1.2.4-Triazol-, 1.2.3-Thiadiazol-, 1.3.4-Thiadiazol-, 1.2.4-Thiadiazol-, 1.2.5-Thiadiazol-, 1.2.3-Oxadiazol-, 1.3.4-Oxadiazol-, 1.2.4-Oxadiazol-, 1.2.5-Oxadiazol-, 1.2.3-Dithiazol-, 1.3.4-Dithiazol-, 1.2.4-Dithiazol- oder Tetrazolring, sowie deren Dihydro- und Tetrahydro-Derivate darstellt, |
| $R_1$-$R_7$ | jeweils unabhängig voneinander Wasserstoff oder $C_1$- $C_5$-Alkyl, |
| X | Wasserstoff, OH oder die Gruppe $-NR_8R_9$, wobei $R_8$ und $R_9$ jeweils unabhängig voneinander Wasserstoff oder $C_1$-$C_5$-Alkyl darstellen, |
| m und n | unabhängig voneinander 0, 1 oder 2, |

bedeuten, sowie deren pharmakologisch unbedenkliche Salze.

Bevorzugt sind hierbei der Pyrazol-, Imidazol-, Thiazol- und 1.2.5-Thiadiazolring, insbesondere der Imidazol- und 4,5-Dihydroimidazolring.

Die heterocyclischen Ringe können gegebenenfalls ein- oder zweifach durch $C_1$-$C_6$-Alkylgruppen, vorzugsweise die Methyl, Ethyl oder Isopropylgruppe, sowie durch Halogen, die Amino- bzw. $CF_3$-Gruppe substituiert sein.

Unter Halogen versteht man Fluor, Chlor oder Brom.

$C_1$-$C_5$-Alkyl soll vorzugsweise die Methyl, Ethyl oder Isopropylgruppe darstellen. Bei der Gruppe $-NR_8R_9$ handelt es sich bevorzugt um die Amino-, Dimethylamino- bzw. Diethylaminogruppe.

X stellt vorzugsweise Wasserstoff oder Hydroxy dar,

n nimmt vor allem den Wert 0 an.

Asymmetrische Kohlenstoffatome können die R- oder S-Konfiguration besitzen und die Verbindungen können in optisch aktiver Form oder als racemisches Gemisch vorliegen. Sie sind ebenfalls Gegenstand der Erfindung. Die enantiomeren Derivate können durch Verwendung optisch reiner Vorstufen erhalten werden.

Verbindungen der allgemeinen Formel I werden nach an sich bekannten Verfahren dargestellt, vorzugsweise in dem man

I. für den Fall, daß X in der allgemeinen Formel I OH bedeutet,

a) eine Carbonsäure der allgemeinen Formel II

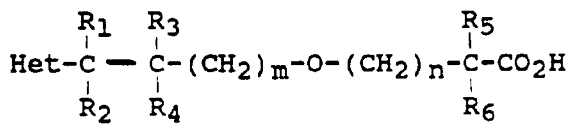 (II),

in der Het, $R_1$-$R_6$, m und n die oben angegebenen Bedeutungen haben, mit einem Gemisch aus phosphoriger Säure oder Phosphorsäure und einem Phosphorhalogenid bzw. Phosphoroxyhalogenid umsetzt und anschließend zur freien Diphosphonsäure verseift,

oder

b) ein Carbonsäurechlorid der allgemeinen Formel III

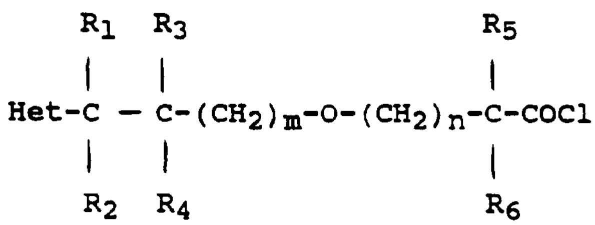 (III),

in der Het, $R_1$-$R_6$, m und n die oben genannten Bedeutungen haben, mit einem Trialkylphosphit der allgemeinen Formel IV

$P(OR')_3$ (IV),

in der R' für Alkylreste mit 1-4 Kohlenstoffatomen, vorzugsweise Methyl, Ethyl, Isopropyl und Isobutyl steht, zu einem Acylphosphonat der allgemeinen Formel V

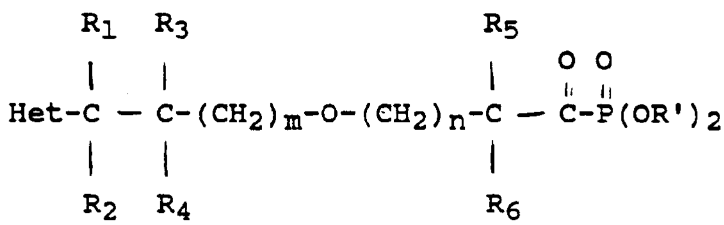

in der Het, $R_1$-$R_6$, m, n und R' die oben genannten Bedeutungen haben, umsetzt, anschließend mit einem Dialkylphosphit der allgemeinen Formel VI

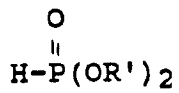 (VI),

in der R' die oben angegebene Bedeutung hat, zu einem Diphosphonat der allgemeinen Formel VII

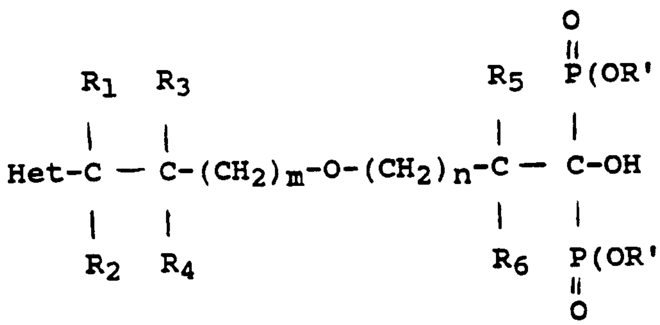 (VII),

in der Het, $R_1$-$R_6$, m, n, und R' die oben angebenen Bedeutungen haben, reagieren läßt und gegebenenfalls die entstandenen Tetraester zu Diestern oder Säuren der allgemeinen Formel I verseift, oder

c) für den Fall, daß n = O bedeutet, eine Verbindung der allgemeinen Formel VIII

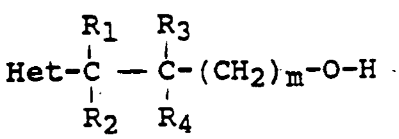 (VIII),

in der Het, $R_1$-$R_4$ und m die oben angegebenen Bedeutungen haben mit einem Epoxid der allgemeinen Formel IX

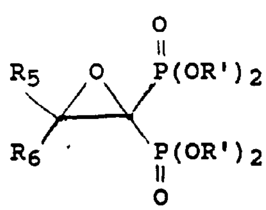 (IX),

in der $R_5$, $R_6$ und R' die oben angegebenen Bedeutungen haben, reagieren läßt und das entstandene Diphosphonsäurederivat der allgemeinen Formel X

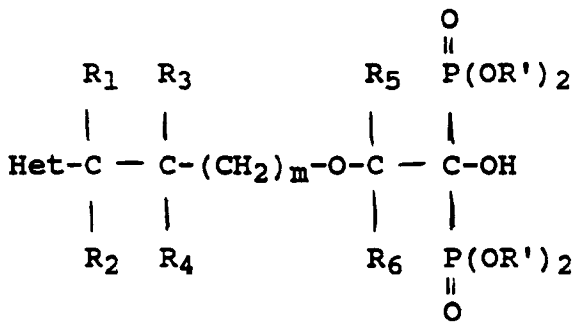 (X),

gewünschtenfalls zu Diestern oder Säuren verseift,

oder

II. für den Fall, daß X in der allgemeinen Formel I die Gruppe $-NR_8R_9$ bedeutet, ein Carbonsäurederivat der allgemeinen Formel XI

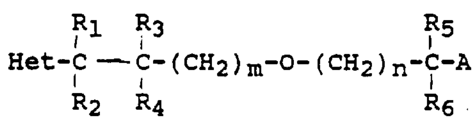 (XI),

in der Het, $R_1$-$R_6$, m und n die oben angegebenen Bedeutungen haben und A eine Nitril-, Iminoether- oder eine $-CONR_8R_9$-Gruppe, wobei $R_8$ und $R_9$ die oben angegebenen Bedeutungen haben, darstellt, mit einer Phosphorverbindung der allgemeinen Formel XII

$$PT_3 \qquad (XII),$$

in der T = Halogen, OH oder OR' bedeutet, wobei R' die oben angegebene Bedeutung hat, umsetzt und gegebenenfalls anschließend verseift,

oder

III. für den Fall, daß X in der allgemeinen Formel I Wasserstoff bedeutet,

a) eine Verbindung der allgemeinen Formel XIII

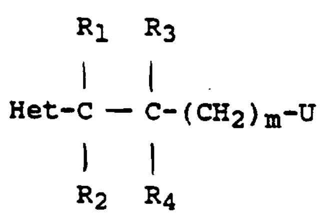 (XIII),

in der Het, $R_1$-$R_4$ und m die oben angegebenen Bedeutungen haben und U eine reaktive Gruppe wie z.B. Halogen oder ein Sulfonat darstellt, mit einem Diphosphonsäurederivat der allgemeinen Formel XIV,

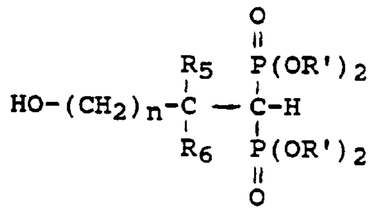

(XIV),

in der $R_5$, $R_6$, R'und n die oben angegebenen Bedeutungen haben, reagieren läßt und die gebildeten Tetraester gegebenenfalls zu Diestern oder Säuren verseift,
oder
b) eine Verbindung der allgemeinen Formel VIII

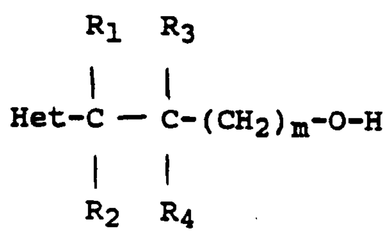

(VIII),

in der Het, $R_1$-$R_4$, und m die oben angegebenen Bedeutungen haben, an eine Verbindung der allgemeinen Formel XV

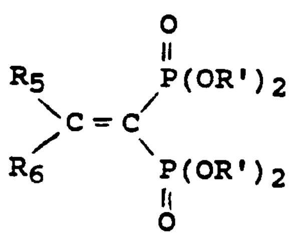

(XV),

in der $R_5$, $R_5$ und R' die oben angegebenen Bedeutungen haben, addiert und die entstehenden Tetraester gegebenenfalls zu Diestern oder Säuren verseift,
oder
c) eine Verbindung der allgemeinen Formel XVI

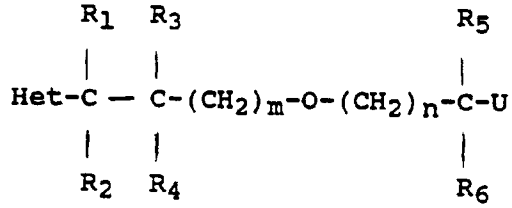

(XVI),

in der Het, $R_1$-$R_6$, U, m und n die oben angegebenen Bedeutungen haben, mit einem Diphosphonsäurederivat der allgemeinen Formel XVII

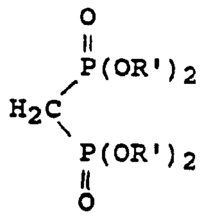

(XVII),

in der R' die oben angegebene Bedeutung hat, umsetzt und die entstandenen Tetraester gegebenenfalls zu Diestern oder Säuren verseift,
oder
für den Fall, daß $R_6$ Wasserstoff bedeutet,
d) eine Verbindung der allgemeinen Formel XVIII

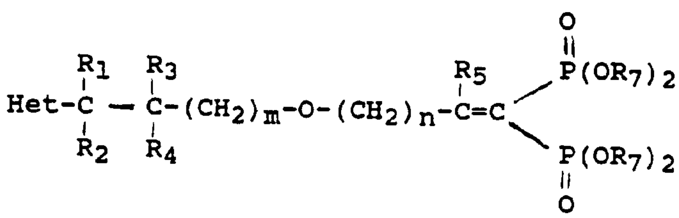

(XVIII),

in der Het, $R_1$-$R_5$ und $R_7$, m und n die oben angegebenen Bedeutungen haben, katalytisch hydriert und anschließend gegebenenfalls die entstandenen Tetraester zu Diestern oder Säuren verseift und gewünschtenfalls in pharmakologisch verträgliche Salze überführt.

Die bei Verfahren I a) eingesetzten Carbonsäuren der allgemeinen Formel II werden mit 1-2, vorzugsweise 1.5 mol phosphoriger Säure oder Phosphorsäure und 1-2, vorzugsweise 1.5 mol Phosphortrihalogenid oder Phosphoroxyhalogenid bei Temperaturen von 80-130°C, vorzugsweise 100-110°C umgesetzt. Man kann die Reaktion auch in Gegenwart von Verdünnungsmitteln wie Halogenkohlenwasserstoffen, insbesondere Chlorbenzol, Tetrachlorethan oder auch Sulfolan bzw. Dioxan durchführen. Die anschließende Hydrolyse erfolgt durch Kochen mit Wasser, zweckmäßigerweise jedoch mit halbkonzentrierter Salz- oder Bromwasserstoffsäure. Als Phosphortrihalogenide kommen in dem genannten Verfahren beispielsweise Phosphortrichlorid oder Phosphortribromid, als Phosphoroxyhalogenid vor allem Phosphoroxychlorid infrage.

Bei Verfahren I b) läßt man das Säurechlorid der allgemeinen Formel III mit dem Trialkylphosphit der allgemeinen Formel IV bei Temperaturen zwischen 0 und 60°C, vorzugsweise bei 20-40°C zur Reaktion kommen. Man kann ohne Lösungsmittel oder auch in Gegenwart von inerten Lösungsmitteln wie Diethylether, Tetrahydrofuran, Dioxan oder auch halogenierten Kohlenwasserstoffen, wie z.B. Methylenchlorid arbeiten. Das als Zwischenprodukt entstehende Acylphosphonat der allgemeinen Formel V kann isoliert oder direkt weiter umgesetzt werden. Die anschließende Reaktion führt man in Gegenwart einer schwachen Base, vorzugsweise einem sec. Amin wie z.B. Dibutylamin bei einer Temperatur von 0-60°C, vorzugsweise bei 10-30°C durch. Die saure Hydrolyse kann sehr gut durch Kochen mit halbkonzentrierter Salzsäure oder Bromwasserstoffsäure durchgeführt werden.

Bei Verfahren I c werden die Alkohole der allgemeinen Formel VIII in der Regel in Form ihrer Alkalisalze, vorzugsweise als Natriumsalze eingesetzt. Als Lösungsmittel verwendet man bevorzugt Toluol, Dioxan, Tetrahydrofuran oder auch Dimethylformamid; die Reaktionen werden zwischen 20 und 80°C durchgeführt.

Bei Verfahren II setzt man die Nitrile der allgemeinen Formel XI mit phosphoriger Säure bei Temperaturen von 110-180°C um. Die Reaktion kann ohne oder in Gegenwart von aprotischen Lösungsmitteln wie z.B. Diethylenglykoldimethylether oder Diethylenglykoldiethylether durchgeführt werden. Man kann die Nitrile jedoch auch mit einem Phosphortrihalogenid, z.B. Phosphortribromid oder Phosphortrichlorid in einem inerten Lösungsmittel wie z.B. Dioxan oder Tetrahydrofuran gegebenenfalls unter Zusatz von Wasser bei Temperaturen von 20-80°C zur Reaktion bringen. Iminoether der allgemeinen Formel XI läßt man mit Dialkylphosphiten vorzugsweise in Gegenwart äquimolarer Mengen Natrium in inerten Lösungsmitteln wie Diethylether, Dioxan oder auch Benzol reagieren, wobei die Umsetzungen in der Regel bei der Rückflußtemperatur des entsprechenden Lösungsmittels stattfinden. Saureamide der allgemeinen Formel XI kann man in inerten Lösungsmitteln wie z.B. halogenierten Kohlenwasserstoffen oder Ethern wie z.B. Diethylether mit einem Gemisch aus Phosphorpentahalogenid/phosphoriger Säure oder auch Oxalylchlorid/Trialkylphosphit umsetzen.

Bei Verfahren III a) setzt man das Diphosphonsäurederivat der allgemeinen Formel XIV in Form eines Natrium- oder Kaliumsalzes ein. Hierzu wird es mit Natrium, Kalium oder dem entsprechenden Hydrid in einem inerten Lösungsmittel wie z.B. Benzol, Toluol oder Dimethylformamid bei einer Temperatur von 0 bis 40°C, vorzugsweise bei 25°C umgesetzt. Das Alkalisalz wird ohne Isolierung mit dem entsprechenden Halogenid bzw. Sulfonat zur Reaktion gebracht. Die Temperatur liegt hierbei bei 20-110°C.

Bei Verfahren III b werden die Alkohole der allgemeinen Formel VIII in Form ihrer Alkalisalze, vorzugsweise der Natriumsalze eingesetzt. Hierzu werden sie mit Natrium bzw. Natriumhydrid in einem inerten Lösungsmittel wie Benzol, Toluol, Dioxan oder Dimethylformamid bei einer Temperatur von 0-60°C, vorzugsweise bei 25°C umgesetzt. Das Alkalisalz wird in der Regel ohne Isolierung mit dem entsprechenden Diphosphonat der allgemeinen Formel XV zur Reaktion gebracht. Die Temperatur liegt bei 20-80°C.

Bei Verfahren III c) setzt man den Methandiphosphonsäureester der allgemeinen Formel XVII in Form seines Natrium- oder Kaliumsalzes ein. Hierzu wird er mit Natrium, Kalium oder dem entsprechenden Hydrid in einem inerten Lösungsmittel wie z.B. Benzol, Toluol oder Dimethylformamid bei einer Temperatur von 0 bis 40°C, vorzugsweise bei 25°C umgesetzt. Das Alkalisalz wird ohne Isolierung mit dem entsprechenden Halogenid bzw. Sulfonat zur Reaktion gebracht. Die Temperatur liegt hierbei bei 20-110°C.

Die Hydrierung bei Verfahren III d wird in Gegenwart eines Edelmetallkatalysators wie z.B. Palladium auf Kohle oder Platin in einem Alkohol wie Methanol oder Ethanol als Lösungsmittel oder auch in Wasser durchgeführt. Man kann jedoch auch Nickel in alkalischem Medium verwenden.

Optisch aktive Verbindungen der Formel I werden in der Regel in der Weise hergestellt, daß man optisch aktive Ausgangsverbindungen einsetzt.

Die bei Verfahren I a eingesetzten Carbonsäuren der Formel II werden in der Regel auf folgende Weise hergestellt.

Das entsprechende Het-alkanol der Formel VIII wird mit einem Halogenalkansäureester, vorzugsweise für den Fall, daß n = 0 ist, mit einem Halogenessigsäureester wie z.B. Brom- oder Chloressigsäureethylester umgesetzt. Der entstandene Carbonsäureester wird nach üblichen Methoden sauer oder alkalisch verseift.

Die bei diesem Verfahren sowie auch Verfahren I c und III b eingesetzten Het-alkanole der Formel VIII sind in der Regel literaturbekannt oder lassen sich aus den entsprechenden Aminosäuren bzw. deren Ester leicht durch Reduktion mit z.B. Lithiumaluminiumhydrid herstellen (s. z.B. Bull.Soc.Chim. France 1969, 2835).

Carbonsäurechloride der allgemeinen Formel III lassen sich nach üblichen Verfahren, z.B. durch Reaktion mit Thionylchlorid oder Phosphorpentachlorid, aus den vorher beschriebenen Carbonsäuren der allgemeinen Formel II herstellen.

Die bei Verfahren 1 c eingesetzten Epoxide der allgemeinen Formel IX sind teilweise literaturbekannt (s. z.B. US-Pat. 3.940.436) oder lassen sich nach den dort angegebenen Methoden herstellen.

Die bei Verfahren II verwendeten Nitrile oder Amide der Formel XI lassen sich aus den entsprechenden Het-alkanolen der Formel VIII durch Umsetzung mit Halogenalkansäurenitrilen bzw. Halogenalkansäureamiden synthetisieren. Aus den so gewonnenen Nitrilen kann man nach üblichen Verfahren, z.B. durch Reaktion mit einem niederen Alkohol in Gegenwart von gasförmigem Chlorwasserstoff die entsprechenden Iminoether erhalten.

Durch Reaktion eines Het-alkanols der Formel VIII mit einem Phosphorhalogenid wie z.B. Phosphortrichlorid oder Phosphortribromid bzw. mit einem aliphatischen oder aromatischen Sulfochlorid, wie z.B. Methansulfochlorid oder Benzolsulfochlorid erhält man die bei Verfahren III a eingesetzten Verbindungen der allgemeinen Formel XIII.

Die bei Verfahren III d eingesetzten Verbindungen der allgemeinen Formel XVIII können z.B. durch Eliminierung einer H-Y-Gruppe hergestellt werden, wobei Y z.B. ein Halogen, vorzugsweise Brom oder Chlor oder eine Acyloxygruppe, wie z.B. die Acetoxy-, gegebenenfalls substituierte Benzoyloxy oder die Trifluoracetoxygruppe, darstellt. Die für die Verbindungen der allgemeinen Formel XVIII eingesetzten Ausgangsmaterialien, wie z.B. die entsprechenden Acyloxyverbindungen, lassen sich aus den Diphosphonaten der allgemeinen Formel I mit X = OH durch Umsetzung mit einem Acylanhydrid wie z.B. Acetanhydrid oder Trifluoracetanhydrid herstellen. Die Umsetzung geschieht in Regel durch Kochen in dem entsprechenden Acylierungsmittel. Die Eliminierung der H-Y-Gruppe kann durch Basen wie z.B. tert.-Amine, insbesondere Triethylamin, Pyridin oder Diazabicycloundecen, in Lösungsmitteln wie Alkoholen, Ethern (z.B. Dioxan oder Tetrahydrofuran) erfolgen. Bei der Abspaltung von Essigsäure, Trifluoressigsäure oder einer gegebenenfalls substituierten Benzoesäure setzt man vorzugsweise das Tetranatrium- oder Tetrakaliumsalz der entsprechenden Diphosphonsäure ein und führt die Abspaltung durch Erhitzen auf 180-300°C, vorzugsweise 180-240°C durch. Als Wärmeüberträger lassen sich dabei sehr gut hoch siedende Amine wie z.B. Ethylanilin oder Collidin einsetzen, die gleichzeitig die freiwerdende Säure als Ammoniumsalz binden. Aus dem Tetraalkalisalz kann man die freien Säuren dann z.B. durch Behandlung mit einem sauren Ionenaustauscher (z.B. Amberlite-IR 120, $H^+$-Form) freisetzen.

Die oben angeführten Ausgangsverbindungen können als Racemate oder als Enantiomere eingesetzt werden, wobei die optisch aktiven Verbindungen üblicherweise aus entsprechend optisch aktiven Hetero-alkancarbonsäuren erhalten werden.

Die bei den Verfahren gegebenenfalls anfallenden Tetraalkylester können zu Diestern oder den freien Tetrasäuren verseift werden. Die Verseifung zu Diestern geschieht in der Regel dadurch, daß man den Tetraalykester mit einem Alkalihalogenid, vorzugsweise Natriumjodid in einem geeigneten Lösungsmittel wie z.B. Aceton bei Zimmertemperatur behandelt.

Hierbei entsteht das symmetrische Diester/Dinatriumsalz, das gegebenenfalls durch einen sauren Ionenaustauscher in die Diester/Disäure umgewandelt werden kann. Die Verseifung zu freien Diphosphonsäuren geschieht in der Regel durch Kochen mit halbkonzentrierter Salz- oder Bromwasserstoffsäure. Man kann jedoch auch eine Spaltung mit Trimethylsilylhalogenid, vorzugsweise dem Bromid oder Jodid vornehmen. Die freien Diphosphonsäuren können umgekehrt durch Kochen mit Orthoameisensäurealkylestern wieder in die Tetraalkylester überführt werden. Die freien Diphosphonsäuren der allgemeinen Formel I können als freie Säuren oder in Form ihrer Mono- oder Dialkalisalze isoliert werden. Die Alkalisalze lassen sich in der Regel durch Umfällen aus Wasser/Methanol oder Wasser/Aceton gut reinigen.

Als pharmakologisch verträgliche Salze werden vor allem Alkalioder Ammoniumsalze verwendet, die man in üblicher Weise z. B. durch Titrieren der Verbindungen mit anorganischen oder organischen Basen wie z.B. Natrium- oder Kaliumhydrogencarbonat, Natronlauge, Kalilauge, wässrigem Ammoniak oder Aminen wie z.B. Trimethyl- oder Triethylamin herstellt.

Die erfindungsgemäßen neuen Substanzen der Formel I und ihre Salze können in flüssiger oder fester Form enteral oder parenteral appliziert werden. Hierbei kommen alle üblichen Applikationsformen infrage, beispielsweise Tabletten, Kapseln, Dragees, Sirupe, Lösungen, Suspensionen etc.. Als Injektionsmedium kommt vorzugsweise Wasser zur Anwendung, welches die bei Injektionslösungen üblichen Zusätze wie Stabilisierungsmittel, Lösungsvermittler und Puffer enthält. Derartige Zusätze sind z.B. Tartrat- und Citrat-Puffer, Ethanol, Komplexbildner (wie Ethylendiamintetraessigsäure und deren nichttoxische Salze), hochmolekulare Polymere (wie flüssiges Polyethylenoxid) zur Viskositätsregelung. Flüssige Trägarstoffe für Injektionslösungen müssen steril sein und werden vorzugsweise in Ampullen abgefüllt. Feste Trägerstoffe sind z.B. Stärke, Lactose, Mannit, Methylcellulose, Talkum, hochdisperse Kieselsäuren, höhermolekulare Fettsäuren (wie Stearinsäure), Gelatine, Agar-Agar, Calciumphosphat, Magnesiumstearat, tierische und pflanzliche Fette, feste hochmolekulare Polymere (wie Polyethylenglykole); für orale Applikation geeignete Zubereitungen können gewünschtenfalls Geschmacks- und Süßstoffe enthalten. Die Dosierung kann von verschiedenen Faktoren, wie Applikationsweise, Spezies, Alter und/oder individuellem Zustand abhängen. Die tägliche zu verabreichenden Dosen liegen bei etwa 0.1-100 mg/ Mensch, vorzugsweise bei 1-20 mg/Mensch und können auf einmal oder mehrere Male verteilt eingenommen werden.

Bevorzugt im Sinne der vorliegenden Erfindung sind außer den in den Beispielen genannten Verbindungen und durch Kombination aller in den Ansprüchen genannten Bedeutungen der Substituenten ableitbaren Verbindungen die folgenden Diphosphonate, sowie deren Natriumsalze, Methyl-, Ethyl- oder Isopropylester:
5-(Imidazol-1-yl)-3-oxa-pentan-1.1-diphosphonsäure
5-(Imidazol-1-yl)-3-oxa-hexan-1.1-diphosphonsäure
S-5-(Imidazol-1-yl)-3-oxa-hexan-1.1-diphosphonsäure
R-5-(Imidazol-1-yl)-3-oxa-hexan-1.1-diphosphonsäure
5-(Imidazol-1-yl)-4-methyl-3-oxa-pentan-1.1-diphosphonsäure
5-(2-Methylimidazol-1-yl)-3-oxa-hexan-1.1-diphosphonsäure
5-(4.5-Dihydro-2-methylimidazol-1-yl)-3-oxa-hexan-1.1-diphosphonsäure
4-(Imidazol-2-yl)-3-oxa-butan-1.1-diphosphonsäure
5-(Imidazol-2-yl)-3-oxa-pentan-1.1-diphosphonsäure
5-(Imidazol-2-yl)-3-oxa-hexan-1.1-diphosphonsäure
5-(Imidazol-2-yl)-4-methyl-3-oxa-pentan-1.1-diphosphonsäure
4-(Imidazol-4-yl)-3-oxa-butan-1.1-diphosphonsäure
5-(Imidazol-4-yl)-3-oxa-pentan-1.1-diphosphonsäure
1-Amino-5-(imidazol-1-yl) -3-oxa-hexan-1.1-diphosphonsäure

1-Dimethylamino-5-(imidazol-1-yl)-3-oxa-hexan-1.1-diphosphonsäure

Die nachfolgenden Beispiele zeigen eine der Verfahrensvarianten, die zur Synthese der erfindungsgemäßen Verbindungen verwendet werden können. Sie sollen jedoch nicht eine Einschränkung des Erfindungsgegenstandes darstellen. Die Verbindungen fallen in der Regel als hochschmelzende Festprodukte an (Mono- oder Dinatriumsalz), deren Struktur durch H-, P- und gegebenenfalls durch $^{13}C$ NMR-Spektroskopie gesichert wurde. Die Reinheit der Substanzen wurde mittels C,H,N,P,S, Na-Analyse sowie durch Dünnschichtelektrophorese (Cellulose, Oxalat-Puffer von pH = 4.0) bestimmt. Zur Charakterisierung der einzelnen Verbindungen werden die $M_{rel}$-Werte (= relative Mobilität) bezogen auf Pyrophosphat ($M_{rel}$ = 1) angegeben.

Beispiel 1

1-Hydroxy-5-(imidazol-1-yl)-3-oxa-pentan-1.1-diphosphonsäure

2.9 g 5-(Imidazol-1-yl)-3-oxa-pentansäure-hydrochlorid (ölige Substanz) werden mit 2.3 g phosphoriger Säure bei 100°C verschmolzen. Man tropft 2.8 ml Phosphortrichlorid zu und hält die Reaktionsmischung 24 Stunden auf 100°C. Nach dem Abkühlen gibt man 25 ml Wasser zu, kocht 1 h unter Rückfluß, filtriert nach dem Abkühlen und engt das Filtrat im Vakuum ein. Den öligen Rückstand löst man in 8 ml Wasser, stellt die Lösung mit ca. 7 ml 10 N Natronlauge auf einen pH = 5 ein und versetzt mit 80 ml Methanol. Nach dem Abkühlen mit Eiswasser fällt ein weißer, schmieriger Niederschlag aus, der in 10 ml Wasser gelöst wird und über 80 g Ionenaustauscher (Amberlite IR-120, $H^+$-Form) gereinigt wird. Man erhält 1.5 g = 32 % der freien Säure, die 1 Mol Wasser enthält; Fp: 125-130°C; $M_{rel}$: 0.30.

Das als Ausgangsmaterial eingesetzte 5-(Imidazol-1-yl)-3-oxa-pentansäure-hydrochlorid wird auf folgende Weise hergestellt: 1-(2-Hydroxyethyl)imidazol (J. Chem. Soc. 1977, 1272) wird in Gegenwart von Natriumhydrid mit Chloressigsäureethylester in Dimethylformamid zum 5-(Imidazol-1-yl)-3-oxa-pentansäureethylester (Öl) umgesetzt und daraus durch Erhitzen mit 6 N Salzsäure die gewünschte Säure gewonnen.

Beistpiel 2

R-1-Hydroxy-5-(imidazol-1-yl)-3-oxa-hexan-1.1-diphosphonsäure

Wie in Beispiel 1 beschrieben erhält man die gewünschte Verbindung aus dem R-5-(Imidazol-1-yl)-3-oxa-hexansäure-hydrochlorid (wachsartige Substanz, $[\alpha]_D^{20}$: -12.4°, c = 1.4 in Wasser) durch Umsetzung mit phosphoriger Säure/Phosphortrichlorid und anschließender Hydrolyse in einer Ausbeute von 27 %. Die Substanz enthält 1 Mol Wasser; $[\alpha]_D^{20}$: -10.2°, c = 1 in Wasser; $M_{rel}$: 0.30

Das als Ausgangsmaterial eingesetzte R-5-(Imidazol-1-yl)-3-oxa-hexansäure-hydrochlorid wird auf folgende Weise hergestellt: R-2-(Imidazol-1-yl)propionsäureethylester (Lieb. Ann. 1986, 327) wird mit Lithiumaluminiumhydrid zum R-2-(Imidazol-1-yl)propanol (ölige Substanz; $[\alpha]_D^{20}$: -17.0°, c = 1 in Methylenchlorid) reduziert, in Gegenwart von Natriumhydrid mit Chloressigsäureethylester in Dimethylformamid zum R-5-(Imidazol-1-yl)-3-oxa-hexansäureethylester (ölige Substanz, $[\alpha]_D^{20}$: -14.8°, c = 1 in Methylenchlorid) umgesetzt und daraus durch Erhitzen mit 6 N Salzsäure die gewünschte Säure gewonnen.

Beispiel 3

In analoger Weise wie in Beispiel 1 beschrieben erhält man durch Umsetzung von Phosphortrichlorid/phosphoriger Säure (nach anschließender Hydrolyse) mit

1.) 6-(Imidazol-1-yl)-3-oxa-hexansäure-hydrochlorid die 1-Hydroxy-6-(imidazol-1-yl)-3-oxa-hexan-1.1-diphosphonsäure

2.) 5-(Imidazol-1-yl)-4-methyl-3-oxa-pentansäure-hydrochlorid die 1-Hydroxy-5-(imidazol-1-yl)-4-methyl-3-oxa-pentan-1.1-diphosphonsäure

3.) S-5-(Imidazol-1-yl)-3-oxa-hexansäure-hydrochlorid die S-1-Hydroxy-5-(imidazol-1-yl)-3-oxa-hexan-1.1-diphosphonsäure

4.) R,S-5-(Imidazol-1-yl)-3-oxa-hexansäure-hydrochlorid die R,S-1-Hydroxy-5-(imidazol-1-yl)-3-oxa-hexan-1.1-diphosphonsäure

5.) 6-(2.4-Dimethylimidazol-1-yl)-3-oxa-hexansäure-hydrochlorid die 6-(2.4-Dimethylimidazol-1-yl)-1-hydroxy-3-oxa-hexan-1.1-diphosphonsäure

6.) 6-(2.5-Dimethylimidazol-1-yl)-3-oxa-hexansäure-hydro- chlorid die 6-(2.5-Dimethylimidazol-1-yl)-1-hydroxy-3-oxa-hexan-1.1-diphosphonsäure

7.) 4-(Imidazol-1-yl)-3-oxa-pentansäure-hydrochlorid die 1-Hydroxy-4-(imidazol-1-yl)-3-oxa-pentan-1.1-diphosphonsäure

8.) 5-(2-Methylimidazol-1-yl)-4-methyl-3-oxa-pentansäure-hydrochlorid die 1-Hydroxy-5-(2-methylimidazol-1-yl)-4-methyl-3-oxa-pentan-1.1-diphosphonsäure

9.) R,S-5-(2-Methylimidazol-1-yl)-3-oxa-hexansäurehydrochlorid die R,S-1-Hydroxy-5-(2-methylimidazol-1-yl)-3-oxa-hexan-1.1-diphosphonsäure

10.) S-5-(2-Methylimidazol-1-yl)-3-oxa-hexansäurehydrochlorid die S-1-Hydroxy-5-(2-methylimidazol-1-yl)-3-oxa-hexan-1.1-diphosphonsäure

11.) R-5-(2-Methylimidazol-1-yl)-3-oxa-hexansäure-hydrochlorid die R-1-Hydroxy-5-(2-methylimidazol-1-yl)-3-oxa-hexan-1.1-diphosphonsäure

12.) 5-(2-Methylimidazol-1-yl)-3-oxa-pentansäurehydrochlorid die 1-Hydroxy-5-(2-methylimidazol-1-yl)-3-oxa-pentan-1.1-diphosphonsäure

13.) (2-Aminoimidazol-1-yl)-3-oxa-pentansäure-hydrochlorid die 5-(2-Aminoimidazol-1-yl)-1-hydroxy-3-oxa-pentan-1.1-diphosphonsäure

14.) 4-(Imidazol-1-yl)-3-oxa-buttersäure-hydrochlorid die 1-Hydroxy-4-(imidazol-1-yl)-3-oxa-butan-1.1-diphosphonsäure

15.) 7-(Imidazol-1-yl)-3-oxa-heptansäure-hydrochlorid die 1-Hydroxy-7-(imidazol-1-yl)-3-oxa-heptan-1.1-diphosphonsäure

16.) 7-(2-Methylimidazol-1-yl)-3-oxa-heptansäure-hydrochlorid die 1-Hydroxy-7-(2-methylimidazol-1-yl)-3-oxa-heptan-1.1-diphosphonsäure

17.) 5-(4.5-Dihydroimidazol-1-yl)-3-oxa-pentansäure-hydrochlorid die 5-(4.5-Dihydroimidazol-1-yl)-1-hydroxy-3-oxa-pentan-1.1-diphosphonsäure

18.) 5-(4.5-Dihydro-2-methylimidazol-1-yl)-3-oxa-hexansäure-hydrochlorid die 5-(4.5-Dihydro-2-methylimidazol-1-yl)-1-hydroxy-3-oxa -hexan-1.1-diphosphonsäure

19.) 5-(4.5-Dihydroimidazol-1-yl)-3-oxa-hexansäure-hydrochlorid die 5-(4.5-Dihydroimidazol-1-yl)-1-hydroxy-3-oxa-hexan-1.1-diphosphonsäure

20.) 5-(4.5-Dihydro-2-methylimidazol-1-yl)-4-methyl-3-oxa-pentansäure-hydrochlorid die 5-(4.5-Dihydro-2-methylimidazol-1-yl)-1-hydroxy-4-methyl-3-oxa-pentan-1.1-diphosphonsäure

21.) 4-(Imidazol-2-yl)-3-oxa-buttersäure-hydrochlorid die 1-Hydroxy-4-(imidazol-2-yl)-3-oxa-butan-1.1-diphosphonsäure

22.) 4-(1-Methylimidazol-2-yl)-3-oxa-buttersäure-hydrochlorid die 1-Hydroxy-4-(1-methylimidazol-2-yl)-3-oxa-butan-1.1-diphosphonsäure

23.) 4-(1-Methylimidazol-2-yl)-3-oxa-pentansäure-hydrochlorid die 1-Hydroxy-4-(1-methylimidazol-2-yl)-3-oxa-pentan-1.1-diphosphonsäure

24.) 4-(4-Brom-1-methylimidazol-2-yl)-3-oxa-pentansäure-hydrochlorid die 4-(4-Brom-1-methylimidazol-2-yl)-1-hydroxy-3-oxa-pentan-1.1-diphosphonsäure

25.) 4-(5-Chlor-3-methylimidazol-2-yl)-3-oxa-pentansäure-hydrochlorid die 4-(5-Chlor-3-methylimidazol-2-yl)-1-hydroxy-3-oxa-pentan-1.1-diphosphonsäure

26.) 5-(Imidazol-2-yl)-4-methyl-3-oxa-pentansäure-hydrochlorid die 1-Hydroxy-5-(imidazol-2-yl)-4-methyl-3-oxa-pentan-1.1-diphosphonsäure

27.) R,S-5-(Imidazol-2-yl)-3-oxa-hexansäure-hydrochlorid die R,S-1-Hydroxy-5-(imidazol-2-yl)-3-oxa-hexan-1.1-diphosphonsäure

28.) S-5-(Imidazol-2-yl)-3-oxa-hexansäure-hydrochlorid die S-1-Hydroxy-5-(imidazol-2-yl)-3-oxa-hexan-1.1-diphosphonsäure

29.) R-5-(Imidazol-2-yl)-3-oxa-hexansäure-hydrochlorid die R-1-Hydroxy-5-(imidazol-2-yl)-3-oxa-hexan-1.1-diphosphonsäure

30.) 5-(Imidazol-2-yl)-3-oxa-pentansäure-hydrochlorid die 1-Hydroxy-5-(imidazol-2-yl)-3-oxa-pentan-1.1-diphosphonsäure

31.) 5-(1-Methylimidazol-2-yl)-3-oxa-pentansäure-hydrochlorid die 1-Hydroxy-5-(1-methylimidazol-2-yl)-3-oxa-pentan-1.1-diphosphonsäure

32.) 5-(Imidazol-2-yl)-5-methyl-3-oxa-hexansäure-hydrochlorid die 1-Hydroxy-5-(imidazol-2-yl)-5-methyl-3-oxa-hexan-1.1-diphosphonsäure

33.) 4-(Imidazol-4-yl)-3-oxa-buttersäure-hydrochlorid die 1-Hydroxy-4-(imidazol-4-yl)-3-oxa-butan-1.1-diphosphonsäure

34.) 4-(5-Methylimidazol-4-yl)-3-oxa-buttersäure-hydrochlorid die 1-Hydroxy-4-(5-methylimidazol-4-yl)-3-oxa-butan-1.1-diphosphonsäure

35.) 4-(5-Trifluormethylimidazol-4-yl)-3-oxa-buttersäure- hydrochlorid die 1-Hydroxy-4-(5-trifluormethylimidazol-4-yl)-3-oxa-butan-1.1-diphosphonsäure

36.) 4-(2-Methylimidazol-4-yl)-3-oxa-buttersäure-hydrochlorid die 1-Hydroxy-4-(2-methylimidazol-4-yl)-3-oxa-butan-1.1-diphosphonsäure

37.) 4-(2-Aminoimidazol-4-yl)-3-oxa-buttersäure-hydrochlorid die 4-(2-Aminoimidazol-4-yl)-1-hydroxy-3-oxa-butan-1.1-diphosphonsäure

38.) 4-(5-Fluorimidazol-4-yl)-3-oxa-buttersäure-hydrochlorid die 4-(5-Fluorimidazol-4-yl)-1-hydroxy-3-oxa-butan-1.1-diphosphonsäure

39.) 4-(Imidazol-4-yl)-3-oxa-pentansäure-hydrochlorid die 1-Hydroxy-4-(imidazol-4-yl)-3-oxa-pentan-1.1-diphosphonsäure

40.) 4-(1-Methylimidazol-4-yl)-3-oxa-pentansäure-hydrochlorid die 1-Hydroxy-4-(1-methylimidazol-4-yl)-3-oxa-pentan-1.1-diphosphonsäure

41.) 5-(Imidazol-4-yl)-3-oxa-pentansäure-hydrochlorid die 1-Hydroxy-5-(imidazol-4-yl)-3-oxa-pentan-1.1-diphosphonsäure

42.) 5-(2-Methylimidazol-4-yl)-3-oxa-pentansäure-hydrochlorid die 1-Hydroxy-5-(2-methylimidazol-4-yl)-3-oxa-pentan-1.1-diphosphonsäure

43.) 6-(Imidazol-4-yl)-3-oxa-hexansäure-hydrochlorid die 1-Hydroxy-6-(imidazol-4-yl)-3-oxa-hexan-1.1-diphosphonsäure

44.) 6-(2-Methylimidazol-4-yl)-3-oxa-hexansäure-hydrochlorid die 1-Hydroxy-6-(2-methylimidazol-4-yl)-3-oxa-hexan-1.1-diphosphonsäure

45.) 5-(4-Methylimidazol-5-yl)-3-oxa-pentansäure-hydrochlorid die 1-Hydroxy-5-(4-methylimidazol-5-yl)-3-oxa-pentan-1.1-diphosphonsäure

46.) 5-(Imidazol-1-yl)-2-methyl-3-oxa-pentansäure-hydrochlorid die 1-Hydroxy-5-(imidazol-1-yl)-2-methyl-3-oxa-pentan-1.1-diphosphonsäure

47.) 5-(Imidazol-1-yl)-2-methyl-3-oxa-hexansäure-hydrochlorid die 1-Hydroxy-5-(imidazol-1-yl)-2-methyl-3-oxa-hexan-1.1-diphosphonsäure

Beispiel 4

Wie in Beispiel 1 angegeben erhält man durch Umsetzung von Brom- bzw. Chloressigsäureethylester oder alpha-Brom- bzw. alpha-Chlorpropionsäureethylester (nach anschließender Verseifung mit 2 N Salzsäure) mit

1.) 1-(3-Hydroxypropyl)imidazol (hergestellt durch Reduktion von 3-(Imidazol-1-yl)propionsäureethylester (Bull.Soc.Chim. France 1969, 2835) mit Lithiumaluminiumhydrid) das 6-(Imidazol-1-yl)-3-oxa-hexansäure-hydrochlorid

2.) 1-(2-Hydroxypropyl)imidazol (J.Chem.Soc. PT 1, 1976, 545) das 5-(Imidazol-1-yl)-4-methyl-3-oxa-pentansäure-hydrochlorid

3.) S-1-(1-Hydroxyprop-2-yl)imidazol (s. Literatur in Beispiel 2) das S-5-(Imidazol-1-yl)-3-oxa-hexansäure-hydrochlorid

4.) R,S-1-(1-Hydroxyprop-2-yl)imidazol (s. Literatur in Beispiel 2) das R,S-5-(Imidazol-1-yl)-3-oxa-hexansäure-hydrochlorid

5.) 1-(3-Hydroxypropyl)-2.4-dimethylimidazol (C.A. 71: 101773 a) das 6-(2.4-Dimethylimidazol-1-yl)-3-oxa-hexansäure-hydrochlorid

6.) 1-(3-Hydroxypropyl)-2.5-dimethylimidazol (C.A. 71: 101773 a) das 6-(2.5-Dimethylimidazol-1-yl)-3-oxa-hexansäure-hydrochlorid

7.) 1-(1-Hydroxyethyl)imidazol (J.Org.Chem. 1967, 2291) das 4-(Imidazol-1-yl)-3-oxa-pentansäure-hydrochlorid

8.) 1-(2-Hydroxypropyl)-2-methylimidazol das 5-(2-Methylimidazol-1-yl)-4-methyl-3-oxa-pentansäure-hydrochlorid

9.) R,S-1-(1-Hydroxyprop-2-yl)-2-methylimidazol das R,S-5-(2-Methylimidazol-1-yl)-3-oxa-hexansäure-hydrochlorid

10.) S-1-(1-Hydroxyprop-2-yl)-2-methylimidazol das S-5-(2-Methylimidazol-1-yl)-3-oxa-hexansäure-hydrochlorid

11.) R-1-(1-Hydroxyprop-2-yl)-2-methylimidazol das R-5-(2-Methylimidazol-1-yl)-3-oxa-hexansäure-hydrochlorid

12.) 1-(2-Hydroxyethyl)-2-methylimidazol das 5-(2-Methylimidazol-1-yl)-3-oxa-pentansäure-hydrochlorid

13.) 2-Amino-1-(2-hydroxyethyl)imidazol (C.A. 76: 46832 v) das (2-Aminoimidazol-1-yl)-3-oxa-pentansäure-hydrochlorid

14.) 1-Hydroxymethylimidazol das 4-(Imidazol-1-yl)-3-oxa-buttersäure-hydrochlorid

15.) 1-(4-Hydroxybutyl)imidazol (Brit.Pat. Nr. 2.016.452) das 7-(Imidazol-1-yl)-3-oxa-heptansäure-hydrochlorid

16.) 1-(4-Hydroxybutyl)-2-methylimidazol (C.A. 99: P 23617 q) das 7-(2-Methylimidazol-1-yl)-3-oxa-heptansäure-hydrochlorid

17.) 4.5-Dihydro-1-(2-hydroxyethyl)imidazol (C.A. 88: 163432 y) das 5-(4.5-Dihydroimidazol-1-yl)-3-oxa-pentansäure-hydrochlorid

18.) 4.5-Dihydro-1-(1-hydroxyprop-2-yl)-2-methylimidazol das 5-(4.5-Dihydro-2-methylimidazol-1-yl)-3-oxa-hexansäure-hydrochlorid

19.) 4.5-Dihydro-1-(1-hydroxyprop-2-yl)imidazol das 5-(4.5-Dihydroimidazol-1-yl)-3-oxa-hexansäure-hydrochlorid

20.) 4.5-Dihydro-1-(2-hydroxypropyl)-2-methylimidazol das 5-(4.5-Dihydro-2-methylimidazol-1-yl)-4-methyl-3-oxa-pentansäure-hydrochlorid

21.) 2-Hydroxymethylimidazol (J.Am.Chem.Soc. 1949, 383) das 4-(Imidazol-2-yl)-3-oxa-buttersäure-hydrochlorid

22.) 2-Hydroxymethyl-1-methylimidazol das 4-(1-Methylimidazol-2-yl)-3-oxa-buttersäure-hydrochlorid

23.) 2-(1-Hydroxyethyl)-1-methylimidazol das 4-(1-Methylimidazol-2-yl)-3-oxa-pentansäure-hydrochlorid

24.) 4-Brom-2-(1-hydroxyethyl)-1-methylimidazol (J.Org.Chem. 1973, 3762) das 4-(4-Brom-1-methylimidazol-2-yl)-3-oxa-pentansäure-hydrochlorid

25.) 5-Chlor-2-(1-hydroxyethyl)-1-methylimidazol (J.Org.Chem. 1973, 3762) das 4-(5-Chlor-1-methylimidazol-2-yl)-3-oxa-pentansäure-hydrochlorid

26.) 2-(2-Hydroxypropyl)imidazol das 5-(Imidazol-2-yl)-4-methyl-3-oxa-pentansäure-hydrochlorid

27.) R,S-2-(1-Hydroxyprop-2-yl)imidazol das R,S-5-(Imidazol-2-yl)-3-oxa-hexansäure-hydrochlorid

28.) S-2-(1-Hydroxyprop-2-yl)imidazol das S-5-(Imidazol-2-yl)-3-oxa-hexansäure-hydrochlorid

29.) R-2-(1-Hydroxyprop-2-yl)imidazol das R-5-(Imidazol-2-yl)-3-oxa-hexansäure-hydrochlorid

30.) 2-(2-Hydroxyethyl)imidazol das 5-(Imidazol-2-yl)-3-oxa-pentansäure-hydrochlorid

31.) 2-(2-Hydroxyethyl)-1-methylimidazol das 5-(1-Methylimidazol-2-yl)-3-oxa-pentansäure-hydrochlorid

32.) 2-(1-Hydroxy-2-methylprop-2-yl)imidazol (J.Chem.Soc. PT2, 1982, 1511) das 5-(Imidazol-2-yl)-5-methyl-3-oxa-hexansäure-hydrochlorid

33.) 4-Hydroxymethylimidazol (Org.Synthesis 24, 64) das 4-(Imidazol-4-yl)-3-oxa-buttersäure-hydrochlorid

34.) 4-Hydroxymethyl-5-methylimidazol (J.Med.Chem. 1976, 923) das 4-(5-Methylimidazol-4-yl)-3-oxa-buttersäure-hydrochlorid

35.) 4-Hydroxymethyl-5-trifluormethylimidazol das 4-(5-Trifluormethylimidazol-4-yl)-3-oxa-buttersäure-hydrochlorid

36.) 4-Hydroxymethyl-2-methylimidazol (J.Med.Chem. 1976, 923) das 4-(2-Methylimidazol-4-yl)-3-oxa-buttersäure-hydrochlorid

37.) 2-Amino-4-hydroxymethylimidazol das 4-(2-Aminoimidazol-4-yl)-3-oxa-buttersäure-hydrochlorid

38.) 5-Fluor-4-hydroxymethylimidazol (J.Am.Chem.Soc. 1973, 4619) das 4-(5-Fluorimidazol-4-yl)-3-oxa-buttersäure-hydrochlorid

39.) 4-(1-Hydroxyethyl)imidazol (J.Med.Chem. 1977, 721) das 4-(Imidazol-4-yl)-3-oxa-pentansäure-hydrochlorid

40.) 4-(1-Hydroxyethyl)-1-methylimidazol das 4-(1-Methylimidazol-4-yl)-3-oxa-pentansäure-hydrochlorid

41.) 4-(2-Hydroxyethyl)imidazol (Arch.Pharm. 1974, 517) das 5-(Imidazol-4-yl)-3-oxa-pentansäure-hydrochlorid

42.) 4-(2-Hydroxyethyl)-2-methylimidazol das 5-(2-Methylimidazol-4-yl)-3-oxa-pentansäure-hydrochlorid

43.) 4-(3-Hydroxypropyl)imidazol (J.Het.Chem. 1975, 577) das 6-(Imidazol-4-yl)-3-oxa-hexansäure-hydrochlorid

44.) 4-(3-Hydroxypropyl)-2-methylimidazol das 6-(2-Methylimidazol-4-yl)-3-oxa-hexansäure-hydrochlorid

45.) 5-(2-Hydroxyethyl)-4-methylimidazol das 5-(4-Methylimidazol-5-yl)-3-oxa-pentansäure-hydrochlorid

46.) 1-(2-Hydroxyethyl)imidazol (s. Beispiel 1) das 5-(Imidazol-1-yl)-2-methyl-3-oxa-pentansäure-hydrochlorid

47.) 1-(1-Hydroxyprop-2-yl)imidazol (s. Beispiel 2) das 5-(Imidazol-1-yl)-2-methyl-3-oxa-hexansäure-hydrochlorid

Beispiel 5

In analoger Weise wie in Beispiel 1 beschrieben erhält man durch Umsetzung von Phosphortrichlorid/phosphoriger Säure (nach anschließender Hydrolyse) mit

1.) 6-(Imidazol-1-yl)-3-oxa-hexansäure-hydrochlorid (ölige Substanz) die 1-Hydroxy-6-(imidazol-1-yl)-3-oxa-hexan-1.1-diphosphonsäure als freie Säure mit 1 Mol Wasser in einer Ausbeute von 28%; $F_p$: 145-

145°C unter Zersetzung, $M_{rel}$: 0.30.

Das als Ausgangsmaterial eingesetzte 6-(Imidazol-1-yl)-3-oxa-hexansäure-hydrochlorid wird auf folgende Weise hergestellt: 1-Trimethylsilylimidazol ($K_{p20}$: 117-120°C) wird mit Chlorpropionsäuretrimethylsilylester ($K_{p1013}$: 172-175°C) unter gleichzeitiger Verseifung des Esters zur 3-(Imidazol-1-yl)-propionsäure (Fp: 148-150°C) umgesetzt. Reaktion mit Trimethylchlorsilan in Ethanol führt zum entsprechenden Ethylesterhydrochlorid, aus dem mit 2 N Natronlauge der freie 3-(Imidazol-1-yl)-propionsäureethylester (Öl) erhalten wurde. Das nach Reduktion mit Lithiumaluminiumhydrid erhaltene 1-(Imidazol-1-yl)-3-propanol (Öl) läßt man mit Bromessigsäureethylester in Dimethylformamid in Gegenwart von Natriumhydrid zum 6-(Imidazol-1-yl)-3-oxa-hexansäurethylester (Öl) reagieren, der anschließend mit 3 N Salzsäure zur gewünschten Säure verseift wird.

2.) 5-(Imidazol-1-yl)-4-methyl-3-oxa-pentansäure-hydrochlorid (ölige Substanz) die 1-Hydroxy-5-(imidazol-1-yl)-4-methyl-3-oxa-pentan-1.1-diphosphonsäure als freie Säure mit 1 Mol Wasser in einer Ausbeute von 37%; Fp: 135-140°C unter Zersetzung. $M_{rel}$: 0.30.

Das als Ausgangsmaterial eingesetzte 5-(Imidazol-1-yl)-4-methyl-3-oxa-pentansäure-hydrochlorid wurde auf folgende Weise hergestellt: Umsetzung des Imidazol-natriumsalzes mit Propenoxyd ergibt das 2-(Imidazol-1-yl)-1-propanol ($K_{p0,1}$: 147-150°C), aus dem man durch Reaktion mit Bromessigsäureethylester in Dimethylformamid in Gegenwart von Natriumhydrid den 5-(Imidazol-1-yl)-4-methyl-3-oxa-pentansäureethylester (Öl) erhält, der dann mit 3 N Salzsäure zur gewünschten Säure verseift wird.

## Patentansprüche

1. Disphosphonate der allgemeinen Formel I

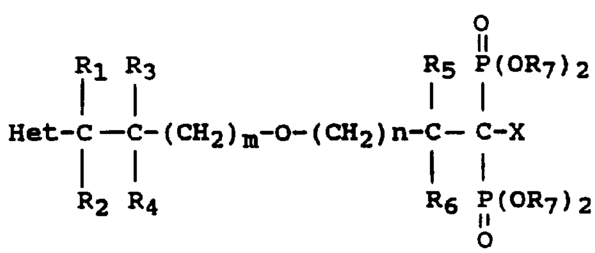

(I),

in der

| | |
|---|---|
| Het | eine Pyrazolyl-, Imidazolyl-, Thiazolyl-, Isothiazolyl-, Oxazolyl-, Isoxazolyl-, Triazolyl-, Thiadiazolyl-, Oxadiazolyl-, Dithiazolyl- oder Tetrazolylring bedeutet, sowie deren Dihydro- und Tetrahydroderivate, der gegebenenfalls ein- oder zweifach durch $C_1$-$C_6$ Alkylgruppen, Halogen, Amino oder $CF_3$ substituiert sein kann |
| $R_1$-$R_7$ | jeweils unabhängig voneinander Wasserstoff oder $C_1$-$C_5$-Alkyl, |
| X | Wasserstoff, OH oder die Gruppe $-NR_8R_9$, wobei $R_8$ und $R_9$ jeweils unabhängig voneinander Wasserstoff oder $C_1$-$C_5$-Alkyl darstellen, |
| m und n | unabhängig voneinander 0, 1 oder 2, |

bedeuten, sowie deren pharmakologisch unbedenkliche Salze, und optisch aktive Formen.

2. Verbindungen der Formel I gemäß Anspruch 1, dadurch gekennzeichnet, daß Het einen Imidazol- oder Dihydroimidazolring bedeutet.

3. Verfahren zur Herstellung von Diphosphonaten der allgemeinen Formel I

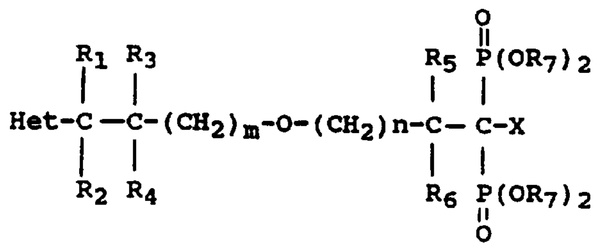 (I),

in der

Het eine Pyrazolyl-, Imidazolyl-, Thiazolyl-, Isothiazolyl-, Oxazolyl-, Isoxazolyl-, Triazolyl-, Thiadiazolyl-, Oxadiazolyl-, Dithiazolyl- oder Tetrazolylring bedeutet, sowie deren Dihydro- und Tetrahydroderivate, der gegebenenfalls ein- oder zweifach durch $C_1$-$C_6$ Alkylgruppen, Halogen, Amino oder $CF_3$ substituiert sein kann,

$R_1$-$R_7$ jeweils unabhängig voneinander Wasserstoff oder $C_1$-$C_5$-Alkyl,

X Wasserstoff, OH oder die Gruppe -$NR_8R_9$, wobei $R_8$ und $R_9$ jeweils unabhängig voneinander Wasserstoff oder $C_1$-$C_5$-Alkyl darstellen,

m und n unabhängig voneinander 0, 1 oder 2,

bedeuten, sowie deren pharmakologisch unbedenkliche Salze, und optisch aktive Formen,
dadurch gekennzeichnet, daß man in an sich bekannter Weise

I. für den Fall, daß X in der allgemeinen Formel I OH bedeutet,

a) eine Carbonsäure der allgemeinen Formel II

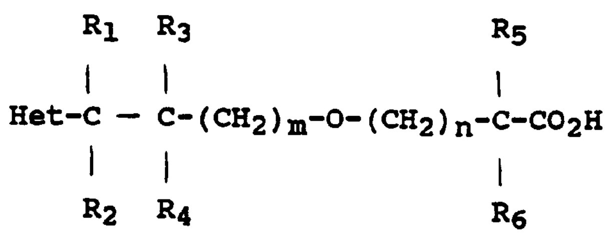 (II),

in der Het, $R_1$-$R_8$, m und n die oben angegebenen Bedeutungen haben, mit einem Gemisch aus phosphoriger Säure oder Phosphorsäure und einem Phosphorhalogenid bzw. Phosphoroxyhalogenid umsetzt und anschließend zur freien Diphosphonsäure verseift,

oder

b) ein Carbonsäurechlorid der allgemeinen Formel III

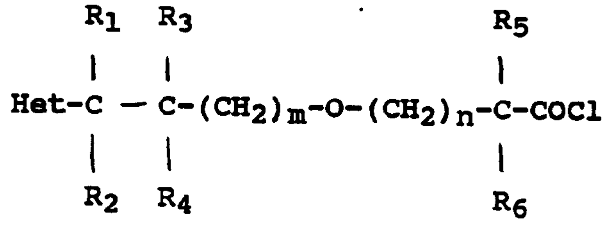 (III),

in der Het, $R_1$-$R_8$, m und n die oben genannten Bedeutungen haben, mit einem Trialkylphosphit der allgemeinen Formel IV

$$P(OR')_3 \quad (IV),$$

in der R' für Alkylreste mit 1-4 Kohlenstoffatomen, vorzugsweise Methyl, Ethyl, Isopropyl und Isobutyl steht, zu einem Acylphosphonat der allgemeinen Formel V

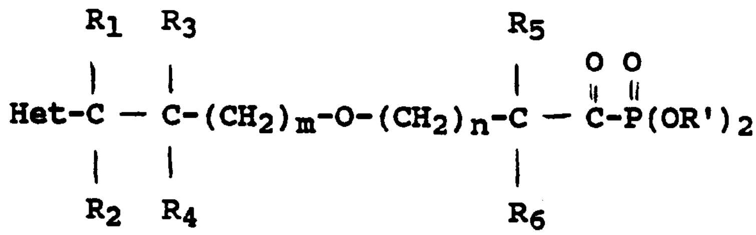

(V),

in der Het, $R_1$-$R_6$, m, n und R' die oben genannten Bedeutungen haben, umsetzt, anschließend mit einem Dialkylphosphit der allgemeinen Formel VI

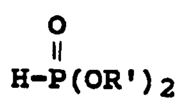

(VI),

in der R' die oben angegebene Bedeutung hat, zu einem Diphosphonat der allgemeinen Formel VII

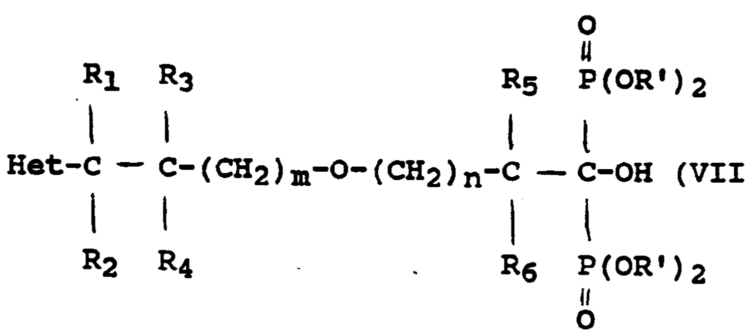

(VII),

in der Het, $R_1$-$R_6$, m, n, und R' die oben angebenen Bedeutungen haben, reagieren läßt und gegebenenfalls die entstandenen Tetraester zu Diestern oder Säuren der allgemeinen Formel I verseift, oder

c) für den Fall, daß n = O bedeutet, eine Verbindung der allgemeinen Formel VIII

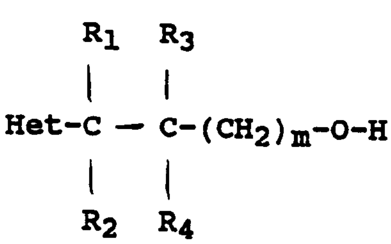

(VIII),

in der Het, $R_1$-$R_4$ und m die oben angegebenen Bedeutungen haben mit einem Epoxid der allge-

meinen Formel IX

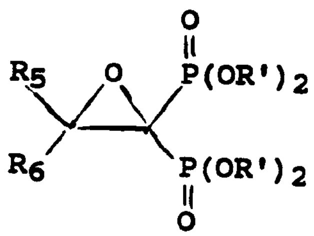

(IX),

in der $R_5$, $R_5$ und R' die oben angegebenen Bedeutungen haben, reagieren läßt und das entstandene Diphosphonsäurederivat der allgemeinen Formel X

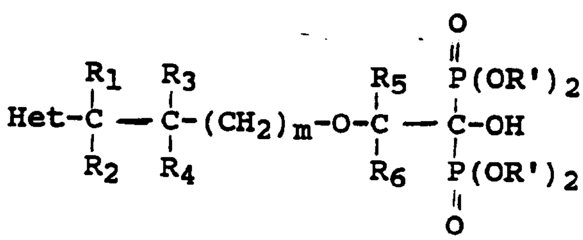

(X),

gewünschtenfalls zu Diestern oder Säuren verseift,
oder

II. für den Fall, daß X in der allgemeinen Formel I die Gruppe $-NR_8R_9$ bedeutet, ein Carbonsäurederivat der allgemeinen Formel XI

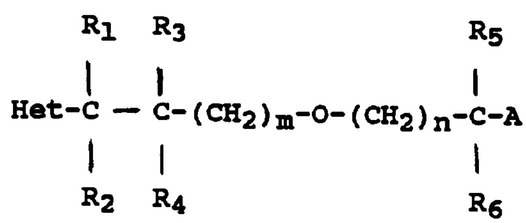

(XI),

in der Het, $R_1$-$R_6$, m und n die oben angegebenen Bedeutungen haben und A eine Nitril-, Iminoether- oder eine $-CONR_8R_9$-Gruppe, wobei $R_5$ und $R_9$ die oben ange-gebenen Bedeutungen haben, darstellt, mit einer Phosphorverbindung der allgemeinen Formel XII

$$PT_3 \qquad (XII),$$

in der T = Halogen, OH oder OR' bedeutet, wobei R' die oben angegebene Bedeutung hat, umsetzt und gegebenenfalls anschließend verseift,
oder

III. für den Fall, daß X in der allgemeinen Formel I Wasserstoff bedeutet,

a) eine Verbindung der allgemeinen Formel XIII

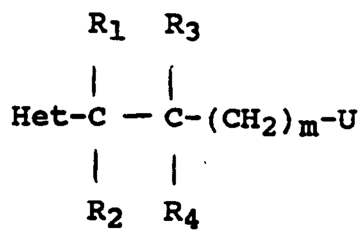

(XIII),

in der Het, $R_1$-$R_4$ und m die oben angegebenen Bedeutungen haben und U eine reaktive Gruppe wie z.B. Halogen oder ein Sulfonat darstellt, mit einem Diphosphonsäurederivat der allgemeinen Formel XIV,

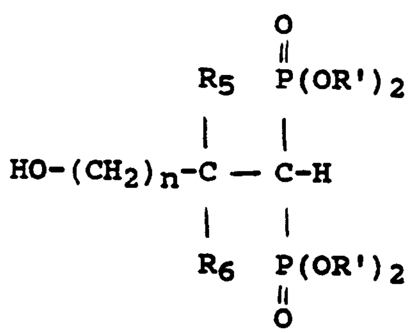

(XIV),

in der $R_5$, $R_6$, R'und n die oben angegebenen Bedeutungen haben, reagieren läßt und die gebildeten Tetraester gegebenenfalls zu Diestern oder Säuren verseift,
oder
b) eine Verbindung der allgemeinen Formel VIII

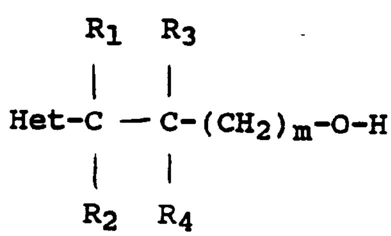

(VIII),

in der Het, $R_1$-$R_4$, und m die oben angegebenen Bedeutungen haben, an eine Verbindung der allgemeinen Formel XV

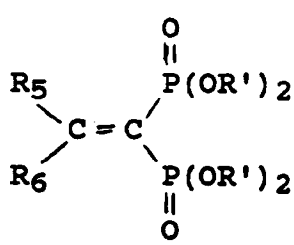

(XV),

in der $R_5$, $R_6$ und R' die oben angegebenen Bedeutungen haben, addiert und die entstehenden Tetraester gegebenenfalls zu Diestern oder Säuren verseift,
oder
c) eine Verbindung der allgemeinen Formel XVI

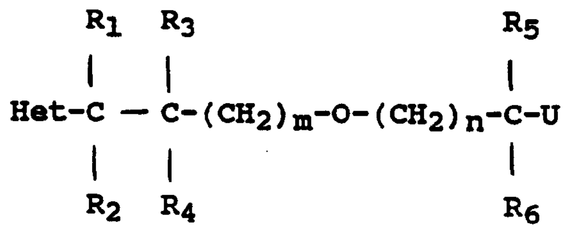

(XVI),

in der Het, $R_1$-$R_6$, U, m und n die oben angegebenen Bedeutungen haben, mit einem Diphosphonsäurederivat der allgemeinen Formel XVII

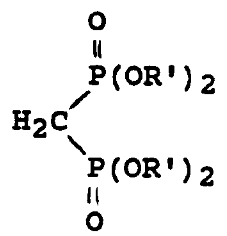

(XVII),

in der R' die oben angegebene Bedeutung hat, umsetzt und die entstandenen Tetraester gegebenenfalls zu Diestern oder Säuren verseift,
oder
für den Fall, daß $R_6$ Wasserstoff bedeutet,
d) eine Verbindung der allgemeinen Formel XVIII

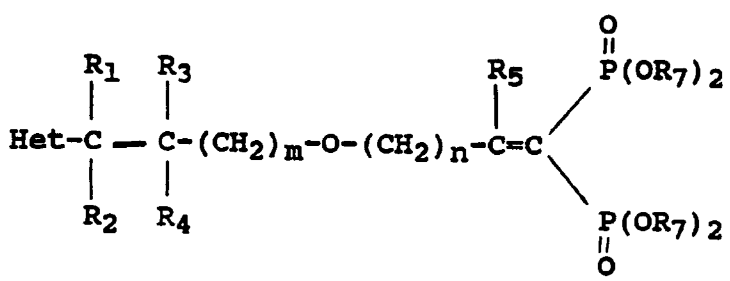

(XVIII),

in der Het, $R_1$-$R_5$ und $R_7$, m und n die oben angegebenen Bedeutungen haben, katalytisch hydriert und anschließend gegebenenfalls die entstandenen Tetraester zu Diestern oder Säuren verseift und gewünschtenfalls in pharmakologisch verträgliche Salze überführt.

**4.** Pharmazeutische Zubereitungen, enthaltend mindestens ein Diphosphonat der allgemeinen Formel I gemäß Anspruch 1 oder 2 neben üblichen pharmazeutischen Hilfs- oder Trägerstoffen.

**5.** Verwendung von Diphosphonaten der allgemeinen Formel I gemäß Anspruch 1 oder 2 zur Herstellung

von Arzneimitteln zur Behandlung von Calciumstoffwechselstörungen.

## Claims

1. Diphosphonates of the general formula I:

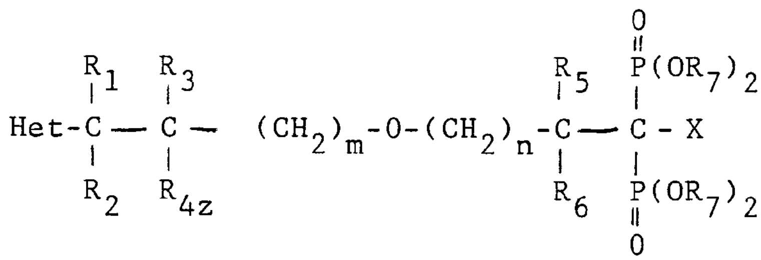

(I),

in which Het signifies a pyrazolyl, imidazolyl, thiazolyl, isothiazolyl, oxazolyl, isoxazolyl, triazolyl, thiadiazolyl, oxadiazolyl, dithiazolyl or tetrazolyl ring, as well as their dihydro and tetrahydro derivatives, which can possibly be substituted once or twice by $C_1$-$C_6$-alkyl groups, halogen, amino or $CF_3$, $R_1$ - $R_7$, in each case independently of one another, hydrogen or $C_1$-$C_5$-alkyl, X hydrogen, OH or the group $-NR_8R_9$, whereby $R_8$ and $R_9$, in each case independently of one another, represent hydrogen or $C_1$-$C_5$-alkyl, m and n, independently of one another, 0, 1 or 2, as well as their pharmacologically acceptable salts and optically-active forms.

2. Compounds of the formula I according to claim 1, characterised in that Het signifies an imidazole or dihydroimidazole ring.

3. Process for the preparation of diphosphonates of the general formula I

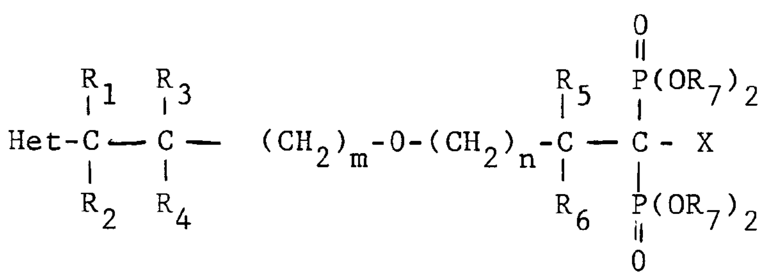

(I)

in which Het signifies a pyrazolyl, imidazolyl, thiazolyl, isothiazolyl, oxazolyl, isoxazolyl, triazolyl, thiadiazolyl, oxadiazolyl, dithiazolyl or tetrazolyl ring, as well as their dihydro and tetrahydro derivatives, which can possibly be substituted once or twice by $C_1$-$C_6$-alkyl groups, halogen, amino or $CF_3$, $R_1$-$R_7$, in each case independently of one another, hydrogen or $C_1$-$C_5$-alkyl, X hydrogen, OH or the group $-NR_8R_9$, whereby $R_8$ and $R_9$, in each case independently of one another, represent hydrogen or $C_1$-$C_5$-alkyl, m and n, independently of one another, 0, 1 or 2, as well as of their pharmacologically acceptable salts and optically-active forms, characterised in that, in per se known manner, one

I. for the case that X in the general formula I signifies OH,

a) reacts a carboxylic acid of the general formula II

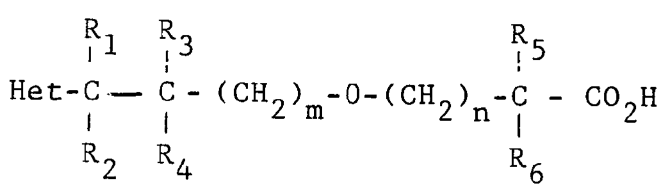

(II),

in which Het, $R_1$-$R_6$, m and n have the above-given meanings, with a mixture of phosphorous acid or phosphoric acid and a phosphorus halide or phosphorus oxyhalide and subsequently saponifies to the free diphosphonic acid or

b) reacts a carboxylic acid chloride of the general formula III

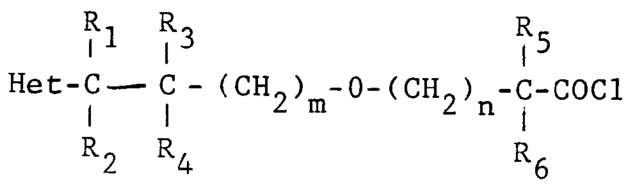

(III)

in which Het, $R_1$-$R_6$, m and n have the above-given meanings, with a trialkyl phosphite of the general formula IV

$P(OR')_3$ (IV),

in which R' stands for an alkyl radical with 1 - 4 carbon atoms, preferably methyl, ethyl, isopropyl and isobutyl, to give an acyl phosphonate of the general formula V

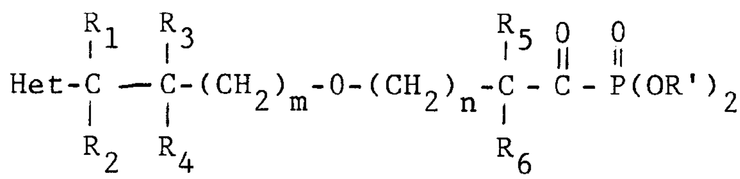

(V),

in which Het, $R_1$-$R_6$, m, n and R' have the above-given meanings, subsequently allows to react with a dialkyl phosphite of the general formula VI

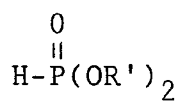

in which R' has the above-given meaning, to give a diphosphonate of the general formula VII

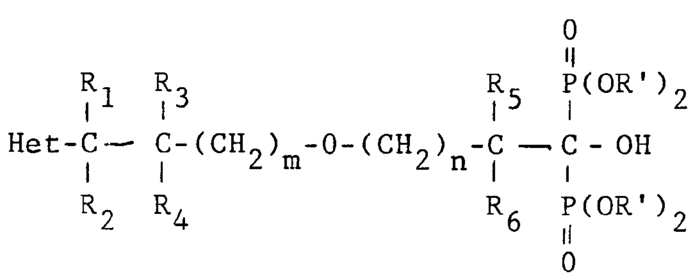

(VII),

in which Het, $R_1$-$R_6$, m, n and R' have the above-given meanings, and possibly saponifies the resulting tetraesters to diesters or acids of the general formula I, or

c) for the case that n = 0, allows a compound of the general formula VIII

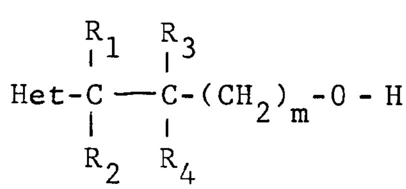

(VIII),

in which Het, $R_1$-$R_4$ and m have the above-given meanings, to react with an epoxide of the general formula IX

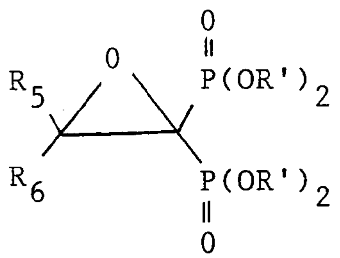

(IX),

in which $R_5$, $R_6$ and R' have the above-given meanings, and, if desired, saponifies the resulting diphosphonic acid derivative of the general formula X

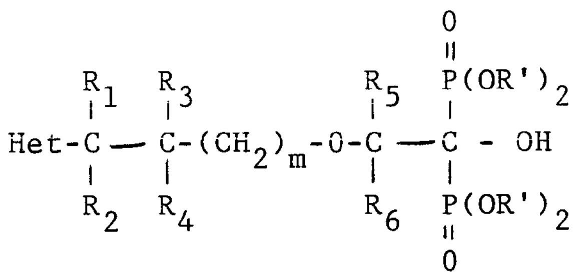

(X),

to diesters or acids, or

II. for the case that X in the general formula I signifies the group $-NR_6R_9$, reacts a carboxylic acid derivative of the general formula XI

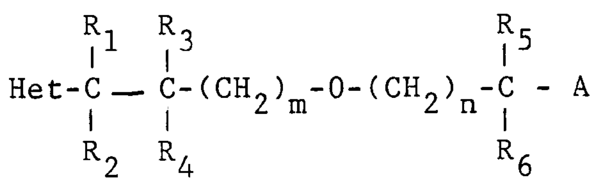

(Xl)

in which Het, $R_1$-$R_6$, m and n have the above-given meanings and A represents a nitrile, iminoether or a $-CONR_8R_9$ group, whereby $R_8$ and $R_9$ have the above-given meanings, with a phosphorus compound of the general formula XII

$PT_3$ (XII)

in which T = halogen, OH or OR', whereby R' has the above-given meaning, and possibly subsequently saponifies, or

III. for the case that X in the general formula I signifies hydrogen,

a) allows a compound of the general formula XIII

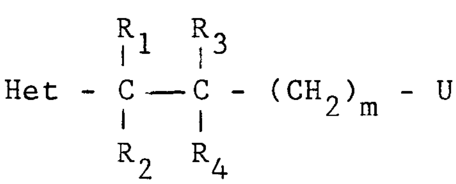

(XIII),

in which Het, $R_1$-$R_4$ and m have the above-given meanings and U represents a reactive group, such

as e.g. halogen or a sulphonate, to react with a diphosphonic acid derivative of the general formula XIV

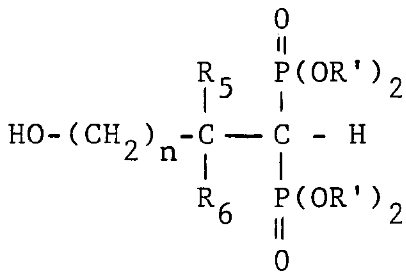

(XIV),

in which $R_5$, $R_6$, R' and n have the above-given meanings, and possibly saponifies the tetraesters formed to diesters or acids, or

b) adds a compound of the general formula VIII

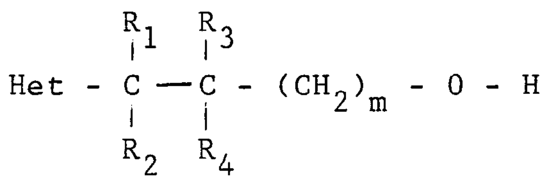

(VIII),

in which Het, $R_1$-$R_4$ and m have the above-given meanings, to a compound of the general formula XV

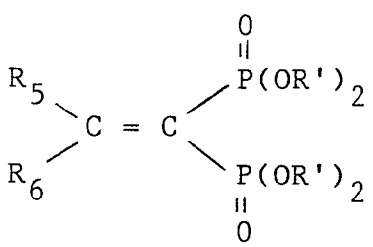

(XV),

in which $R_5$, $R_6$ and R' have the above-given meanings, and possibly saponifies the resultant tetraesters to diesters or acids, or

c) reacts a compound of the general formula XVI

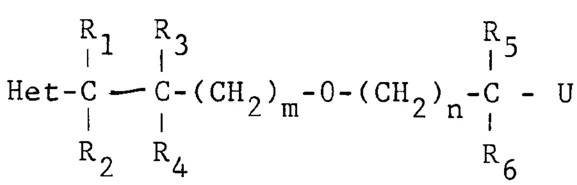

(XVI),

in which Het, $R_1$-$R_6$, U, m and n have the above-given meanings, with a diphosphonic acid derivative of the general formula XVII

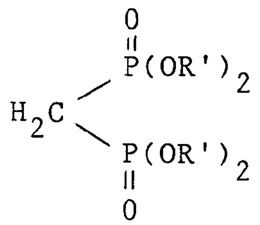

(XVII),

in which R' has the above-given meaning, and possibly saponifies the resultant tetraesters to diesters or acids, or

for the case that $R_6$ signifies hydrogen,

d) catalytically hydrogenates a compound of the general formula XVIII

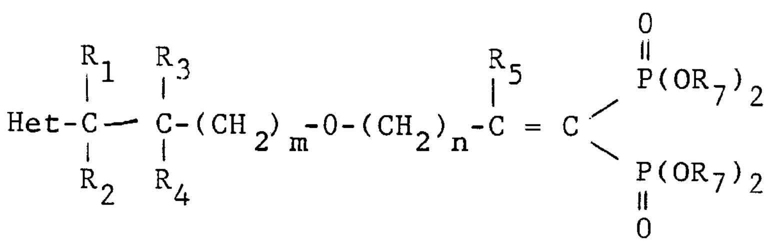

(XVIII)

in which Het, $R_1$-$R_5$ and $R_7$, m and n have the above-given meanings, and subsequently possibly saponifies the resultant tetraesters to diesters or acids and, if desired, converts into pharmacologically acceptable salts.

**4.** Pharmaceutical compositions containing at least one diphosphonate of the general formula I according to claim 1 or 2, besides usual pharmaceutical adjuvant and carrier materials.

**5.** Use of diphosphonates of the general formula I according to claim 1 or 2 for the preparation of medicaments for the treatment of calcium metabolism disturbances.

## Revendications

**1.** Diphosphonates de formule générale I

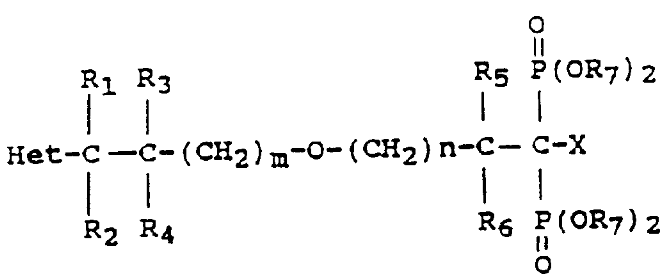

(I),

dans laquelle

| | |
|---|---|
| Het | représente un cycle pyrazolyle, imidazolyle, thiazolyle, isothiazolyle, oxazolyle, isoxazolyle, triazolyle, thiadiazolyle, oxadiazolyle, dithiazolyle ou tétrazolyle, ainsi que leurs dérivés dihydrogénés et tétrahydrogénés, qui peuvent être substitués éventuellement une ou deux fois par un groupe alkyle en $C_1$-$C_6$, un atome d'halogène, un groupe amino ou un groupe $CF_3$, |
| $R_1$-$R_7$ | représentent chacun indépendamment l'un de l'autre un atome d'hydrogène ou un groupe alkyle en $C_1$-$C_5$, |

X représente un atome d'hydrogène, un groupe OH ou le groupe $-NR_8R_9$, dans lequel $R_8$ et $R_9$ représentent chacun, indépendamment l'un de l'autre, un atome d'hydrogène ou un groupe alkyle en $C_1$-$C_3$,

m et n valent, indépendamment l'un de l'autre, 0, 1 ou 2,

ainsi que leurs sels pharmaceutiquement acceptables et leurs formes optiquement actives.

2. Composés de formule générale I, selon la revendication 1, caractérisés en ce que Het représente un cycle imidazole ou dihydroimidazole.

3. Procédé de préparation des diphosphonates de formule générale I

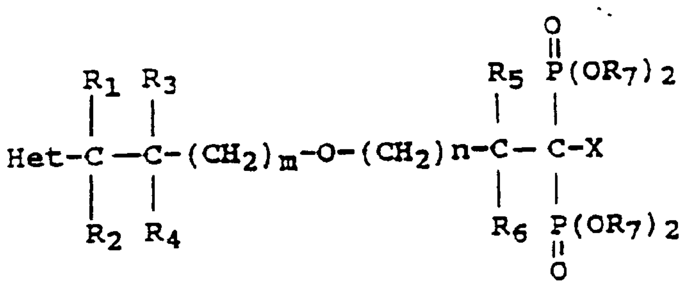

(I),

dans laquelle

Het représente un cycle pyrazolyle, imidazolyle, thiazolyle, isothiazolyle, oxazolyle, isoxazolyle, triazolyle, thiadiazolyle, oxadiazolyle, dithiazolyle ou tétrazolyle, ainsi que leurs dérivés dihydrogénés et tétrahydrogénés, qui peuvent être substitués éventuellement une ou deux fois par un groupe alkyle en $C_1$-$C_6$, un atome d'halogène, un groupe amino ou un groupe $CF_3$,

$R_1$-$R_7$ représentent chacun indépendamment l'un de l'autre un atome d'hydrogène ou un groupe alkyle en $C_1$-$C_5$,

X représente un atome d'hydrogène, un groupe OH ou le groupe $-NR_8R_9$, dans lequel $R_8$ et $R_9$ représentent chacun, indépendamment l'un de l'autre, un atome d'hydrogène ou un groupe alkyle en $C_1$-$C_5$,

m et n valent chacun indépendamment 0, 1 ou 2,

ainsi que leurs sels pharmacologiquement acceptables et leurs formes optiquement actives, caractérisé en ce que, de manière connue en soi, on fait réagir:

I. dans le cas où X dans la formule générale I représente un groupe OH,

a) un acide carboxylique de formule générale II

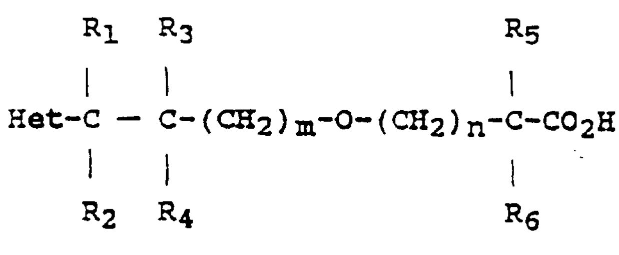

(II),

dans laquelle Het, $R_1$-$R_6$, m et n ont les significations mentionnées ci-dessus, avec un mélange d'acide phosphoreux ou d'acide phosphorique et d'un halogénure de phosphore ou d'un oxyhalogénure de phosphore, et ensuite, on saponifie pour obtenir l'acide diphosphonique libre,

ou

b) on fait réagir un chlorure d'acide carboxylique de formule générale III

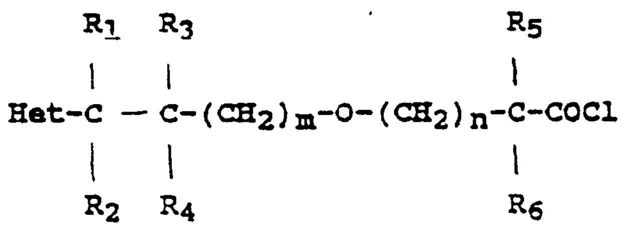

(III),

dans laquelle Het, $R_1$-$R_6$, m et n ont les significations mentionnées ci-dessus, avec un phosphite de trialkyle de formule générale IV

$$P(OR')_3 \qquad (IV)$$

dans laquelle R' représente un groupe alkyle comportant 1-4 atomes de carbone, de préférence un groupe méthyle, éthyle, isopropyle et isobutyle, pour obtenir un phosphonate d'acyle de formule générale V

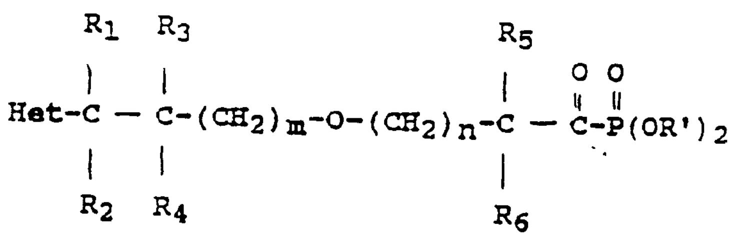

(V),

dans laquelle Het, R1-$R_6$ , m, n et R' ont les significations mentionnées ci-dessus, puis on fait réagir avec un phosphite de dialkyle de formule générale VI

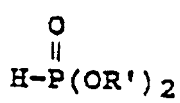

(VI),

dans laquelle R' a la signification mentionnée ci-dessus, pour obtenir un diphosphonate de formule générale VII

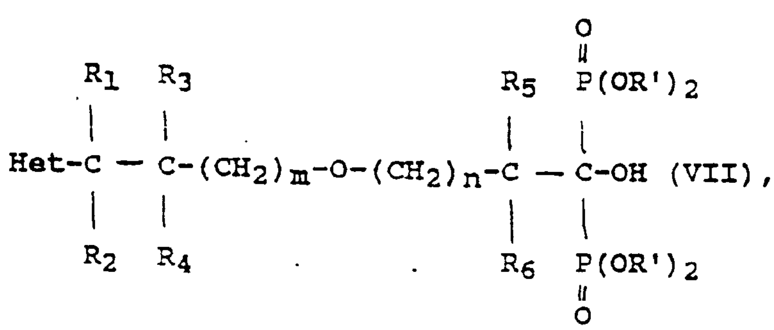

(VII),

dans laquelle Het, $R_1$-$R_6$, m, n et R' ont les significations mentionnées ci-dessus, et éventuellement, on saponifie le tétraester obtenu en diester ou en acide de formule générale I,

ou

c) dans le cas où n = 0, on fait réagir un composé de formule générale VIII

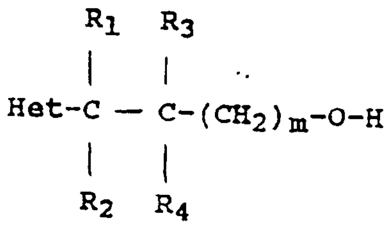

(VIII),

dans laquelle Het, $R_1$-$R_4$ et m ont les significations mentionnées ci-dessus, avec un époxyde de formule générale IX

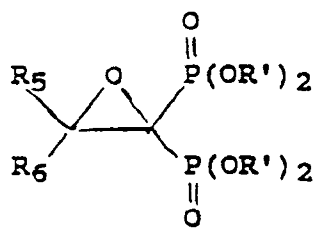

(IX),

dans laquelle $R_5$, $R_6$ et R' ont les significations mentionnées ci-dessus, et l'on saponifie éventuellement le dérivé d'acide diphosphonique obtenu, de formule générale X

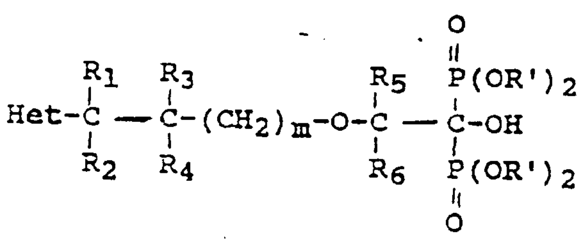

(X),

en diester ou en acide,

ou

II. dans le cas où X dans la formule générale I représente le groupe $-NR_8R_9$,

on fait réagir un dérivé d'acide carboxylique de formule générale XI

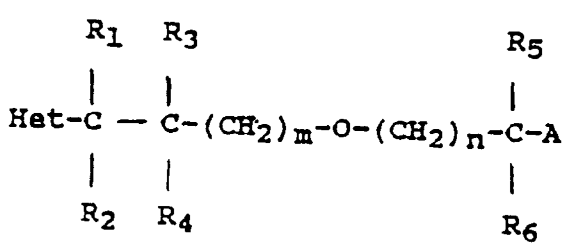

(XI),

dans laquelle Het, $R_1$-$R_6$, m et n ont les significations mentionnées ci-dessus et A représente un groupe nitrile, iminoéther ou $-CONR_8R_9$, dans lequel $R_8$ et $R_9$ ont les significations mentionnées ci-dessus, avec un composé du phosphore de formule générale XII

$PT_3$ (XII),

dans laquelle T = un atome d'halogène, un groupe OH ou OR', dans lequel R' a la signification mentionnée ci-dessus, et éventuellement, on saponifie ensuite,

ou

III. dans le cas où X dans la formule générale I représente un atome d'hydrogène,

a) on fait réagir un composé de formule générale XIII

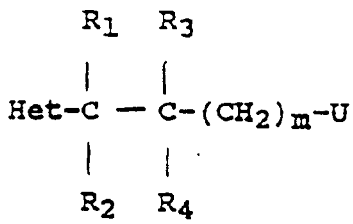

(XIII),

dans laquelle Het, $R_1$-$R_4$ et m ont les significations mentionnées ci-dessus et U représente un groupe réactif tel que par exemple un atome d'halogène ou un groupe sulfonate, avec un dérivé d'acide diphosphonique de formule générale XIV,

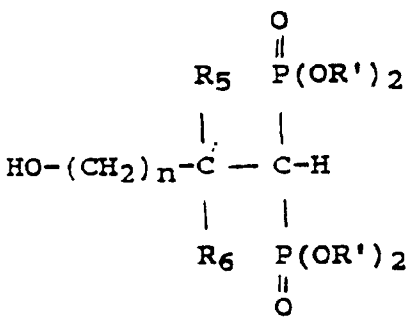

(XIV),

dans laquelle $R_5$, $R_6$, R' et n ont les significations mentionnées ci-dessus, et l'on saponifie éventuellement le tétraester formé en diester ou en acide,

ou

b) on additionne un composé de formule générale VIII

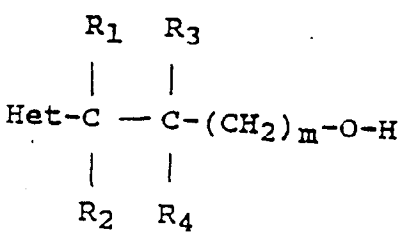

(VIII),

dans laquelle Het, $R_1$-$R_4$ et m ont les significations mentionnées ci-dessus, à un composé de formule générale XV

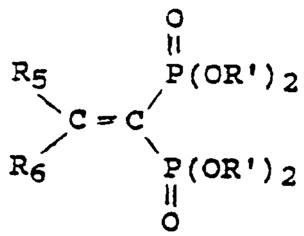

(XV),

dans laquelle $R_5$, $R_6$ et R' ont les significations mentionnées ci-dessus, et l'on saponifie éventuellement le tétraester formé en diester ou en acide,

ou

c) on fait réagir un composé de formule générale XVI

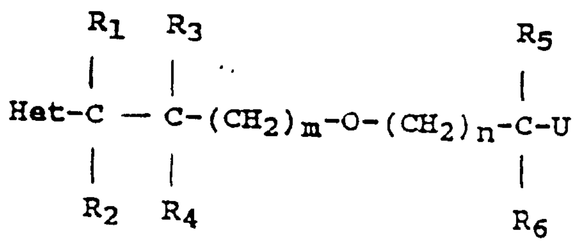

(XVI),

dans laquelle Het, $R_1$-$R_6$, U, m et n ont les significations mentionnées ci-dessus, avec un dérivé d'acide diphosphonique de formule générale XVII

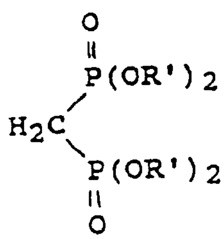

(XVII),

dans laquelle R' a la signification mentionnée ci-dessus, et l'on saponifie éventuellement le tétraester formé en diester ou en acide,

ou

dans le cas où $R_6$ représente un atome d'hydrogène,

d) on soumet un composé de formule générale XVIII

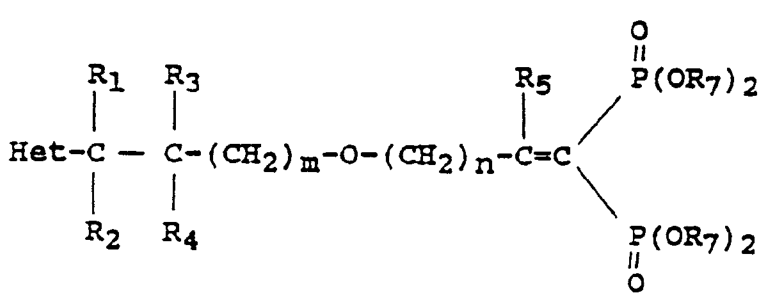

(XVIII),

dans laquelle Het, $R_1$-$R_5$ et $R_7$, m et n ont les significations mentionnées ci-dessus, à une hydrogénation catalytique,
et ensuite, on saponifie éventuellement le tétraester formé en diester ou en acide et transforme éventuellement ces derniers en leurs sels pharmacologiquement acceptables.

**4.** Préparations pharmaceutiques comprenant au moins un diphosphonate de formule générale I selon la revendication 1 ou 2, en plus de véhicules et adjuvants pharmaceutiques usuels.

**5.** Utilisation des diphosphonates de formule générale I selon la revendication 1 ou 2, pour la préparation de médicaments destinés au traitement des troubles du métabolisme calcique.